# EUROPEAN PATENT APPLICATION

(11) **EP 1 281 356 A2**
(43) Date of publication of application: **05.02.2003**
(21) Application number: 02017365.4
(22) Date of filing: 02.08.2002
(51) Int. Cl.: A61B 17/00

(54) **Blood vessel connecting instrument**

(30) Priority: 03.08.2001 JP 2001237088
(71) Applicant: TERUMO KABUSHIKI KAISHA, Tokyo (JP)
(72) Inventor: Kato, Yukitoshi, c/oTerumo Kabushiki Kaisha, Ashigarakami-gun, Kanagawa (JP)
(74) Representative: Casalonga, Axel

(57) **Abstract**

A blood vessel connecting instrument wherein connection of blood vessels to each other can be realized readily, securely and quickly is provided.

A blood vessel connecting instrument 1 for connecting an end portion of a second blood vessel 200 to a side portion of a first blood vessel 100 is provided with a plurality of engaging members 2 constituted by a linear body, a pair of holding members 3, a ring-shaped fastening member 4, a pressing member 5 for pressing and moving the fastening member 4, a blood vessel supporting member 6 supporting the second blood vessel 200, and a receiving member 7 being attached to the second blood vessel 200. An engaging portion 21 is formed on the distal end of each engaging member 2 by bending the linear body substantially at right angles. A peripheral portion 102 of an opening 101 of the first blood vessel 100 can be clipped between the engaging portions 21 and distal end surfaces 32 of the holding members 3.

## Description

### BACKGROUND OF THE INVENTION

### 1. Field of the Invention

The present invention relates to instruments for connecting blood vessels to each other, more particularly to instruments for connecting an end portion of one blood vessel to a side portion of another blood vessel.

### 2. Description of the Related Art

In blood vessel anastomosis operations like a coronary artery bypass graft (CABG) operation, for example, in the case where a bypass between a internal thoracic artery and a left coronary artery is formed, it is carried out by partially dissecting a side portion of the left coronary artery to form an opening and fitting an end portion of the internal thoracic artery to the opening and connecting the peripheral portion of the opening with the end portion by suturing with a suture.

In this case, the success of the suturing operation on the portion of the coronary artery depends on the doctor's skill. Though, it is very difficult to carry out the operation adequately and quickly because the operation is carried out on blood vessels that are very thin and under a narrow field of vision, more particularly under heart beating.

Also, because of the difficulty described above, it takes long time to carry out the operation, it gives much stress on patients.

### SUMMARY OF THE INVENTION

An object of the present invention is to provide a blood vessel connecting instrument which can connect blood vessels to each other easily and quickly.

The above object has been achieved by the following items (1) to (32) according to the present invention.
(1) A blood vessel connecting instrument for connecting an end portion of a second blood vessel to a side portion of a first blood vessel, comprising:
   a plurality of engaging members each having an engaging portion which can be engaged with a peripheral portion of an opening formed in the side portion of the first blood vessel from inside; and
   a holding means for holding the state in which the engaging portion is engaged with the peripheral portion of the opening in the first blood vessel.
(2) A blood vessel connecting instrument according to the item (1) above, which further includes a fastening member for securely tightening the end portion of the second blood vessel overlaid on the vicinity of the peripheral portion of the opening in the first blood vessel.
(3) A blood vessel connecting instrument according to the item (2) above, wherein the fastening member is ring-shaped.
(4) A blood vessel connecting instrument according to the item (2) or (3) above, wherein the fastening member has a variable diameter.
(5) A blood vessel connecting instrument according to any one of the items (2) to (4) above, which further includes a receiving member for receiving fastening force applied by the fastening member.
(6) A blood vessel connecting instrument for connecting an end portion of a second blood vessel to a side portion of a first blood vessel, comprising:
   a plurality of engaging members each having an engaging portion which can be engaged with a peripheral portion of an opening formed in the side portion of the first blood vessel from inside;
   a holding means for holding the state in which the engaging portion is engaged with the peripheral portion of the opening in the first blood vessel;
   a ring-shaped fastening member; and
   a receiving member for receiving fastening force applied by the fastening member,
   wherein the end portion of the second blood vessel is overlaid on the vicinity of the peripheral portion of the opening formed in the first blood vessel, and the overlapping portion of the first blood vessel and the second blood vessel is fastened by the fastening member and the receiving member to connect the end portion of the second blood vessel to the side portion of the first blood vessel.
(7) A blood vessel connecting instrument according to the item (5) or (6) above, wherein the receiving member is ring-shaped.
(8) A blood vessel connecting instrument according to the item (7) above, wherein the receiving member has a groove on its outer periphery.
(9) A blood vessel connecting instrument according to any one of the items (5) to (8) above, which further includes a blood vessel supporting member for supporting the second blood vessel together with the receiving member.
(10) A blood vessel connecting instrument according to the item (9) above, wherein the blood vessel supporting member has a pair of arm portions which can come closer to and spaced from each other.
(11) A blood vessel connecting instrument according to any one of the items (2) to (10) above, which further includes a guide portion for guiding the fastening member to a fixing position in which the first blood vessel is fixed to the second blood vessel and a moving means for moving the fastening member to the fixing position.
(12) A blood vessel connecting instrument according to the item (11) above, wherein the fixing position is remoter from the opening than a position in which the engaging portion is engaged with an inner surface of the first blood vessel.
(13) A blood vessel connecting instrument according to any one of the items (1) to (12) above, wherein each of the engaging members comprises a linear body.
(14) A blood vessel connecting instrument according to the item (13) above, wherein the engaging portion is formed by bending a distal end of the linear body.
(15) A blood vessel connecting instrument according to the item (13) or (14) above, wherein the number of the engaging members is four to six.
(16) A blood vessel connecting instrument according to any one of the items (1) to (15) above, in the plurality of engaging members, at least two of the engaging members are variable with respect to the distance between one engaging member and another one.
(17) A blood vessel connecting instrument according to any one of the items (1) to (16) above, wherein the holding means has a clipping portion so that the peripheral portion of the opening in the first blood vessel can be clipped between the clipping portion and the engaging portion.
(18) A blood vessel connecting instrument according to the item (17) above, wherein the engaging members are arranged in the holding means so as to be movable in the longitudinal direction thereof.
(19) A blood vessel connecting instrument according to the item (17) or (18) above, wherein the holding means has slits through which the second blood vessel can pass.
(20) A blood vessel connecting instrument according to the item (19) above, wherein the holding means comprises a pair of elongated members each having a semi-circular (arch-shaped) cross section and a space between the elongated members placed so that their curved concave surfaces face each other forms the slits.
(21) A blood vessel connecting instrument according to the item (19) above, wherein the holding means is a hollow tube of which both ends are open and has a slit which is a cutout formed on a side surface of the tube.
(22) A blood vessel connecting instrument according to the item (21) above, wherein the holding means has a lumen which has a substantially oval or elliptical shape.
(23) A blood vessel connecting instrument according to the item (22) above, wherein the number of the engaging members provided in a distal end surface of the holding means is four, of which two are arranged in portions corresponding to both ends of the oval or elliptical shape in the long-diameter direction and the other two are arranged in portions corresponding to both ends thereof in the short-diameter direction.
(24) A blood vessel connecting instrument according to any one of the items (21) to (23) above, wherein the slit has a width of one-tenth to one-fourth of the whole peripheral length of the holding means.
(25) A blood vessel connecting instrument for connecting an end portion of a second blood vessel to a side portion of a first blood vessel, comprising:
   a tubular hollow holding member having a slit which is formed in a part of a side surface of the holding member in the peripheral direction and through which the second blood vessel can pass;
   a plurality of engaging members each of which comprises a linear body, is arranged in the holding member so as to be movable in the longitudinal direction thereof, and can be engaged with a peripheral portion of an opening formed in the side portion of the first blood vessel from inside;
   a fastening member; and
   a receiving member for receiving fastening force applied by the fastening member,
   wherein the end portion of the second blood vessel is overlaid on the vicinity of the peripheral portion of the opening in the first blood vessel, and the overlapping portion of the first blood vessel and the second blood vessel is fastened by the fastening member and the receiving member to connect the end portion of the second blood vessel to the side portion of the first blood vessel.
(26) A blood vessel connecting instrument according to the item (25) above, wherein the fastening member or the receiving member or both are ring-shaped.
(27) A blood vessel connecting instrument according to the item (25) or (26) above, wherein the engaging members are inserted into small holes which start from a distal end surface of the holding member and extend in the holding member in the longitudinal direction thereof.
(28) A blood vessel connecting instrument according to any one of the items (25) to (27) above, wherein the distal end surface of the holding member has a substantially oval or elliptical shape.
(29) A blood vessel connecting instrument according to the item (28) above, wherein the number of the engaging members provided in the distal end surface of the holding member is four, of which two are arranged in portions corresponding to both ends of the oval or elliptical shape in the long-diameter direction and the other two are arranged in portions corresponding to both ends thereof in the short-diameter direction.
(30) A blood vessel connecting instrument according to the item (29) above, wherein the slit is formed in a position between an engaging member provided at an end of the distal end surface in the long-diameter direction and an engaging member provided at an end of the distal end surface in the short-diameter direction.
(31) A blood vessel connecting instrument according to any one of the items (25) to (30) above, wherein the slit has a width of one-tenth to one-fourth of the whole peripheral length of the holding member.
(32) A blood vessel connecting instrument according to any one of the items (25) to (31) above, wherein the slit opens on the distal end surface of the holding member.

### BRIEF DESCRIPTION OF THE DRAWINGS

Figs. 1 to 6 each are a longitudinal cross sectional view showing a structure of a blood vessel connecting instrument according to the present invention and using procedure thereof.
Fig. 7 is a perspective view showing a structure of a distal end portion of the blood vessel connecting instrument according to the present invention.
Fig. 8 is a plan view showing how engaging members in the blood vessel connecting instrument of the present invention are attached to an opening (a dissected portion) of a blood vessel.
Fig. 9 is a partial sectional side view showing a structure of a blood vessel supporting member and a receiving member in the blood vessel connecting instrument according to the present invention.
Fig. 10A is a perspective view showing a structure of a fastening member in the blood vessel connecting instrument according to the present invention, illustrating a state of an increased diameter.
Fig. 10B is a perspective view showing a structure of a fastening member in the blood vessel connecting instrument according to the present invention, illustrating a state of a reduced diameter (in an overlapped state).
Fig. 11 is a perspective view showing another structure of the distal end portion of the blood vessel connecting instrument according to the present invention.

### DESCRIPTION OF SOME EMBODIMENTS OF THE INVENTION

Hereinafter, the blood vessel connecting instrument according to various embodiments of the present invention will be illustrated in detail with reference to the attached drawings.

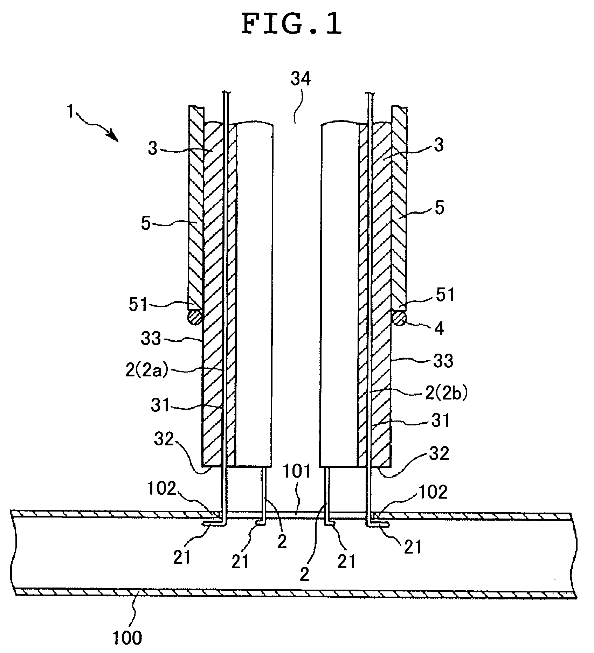

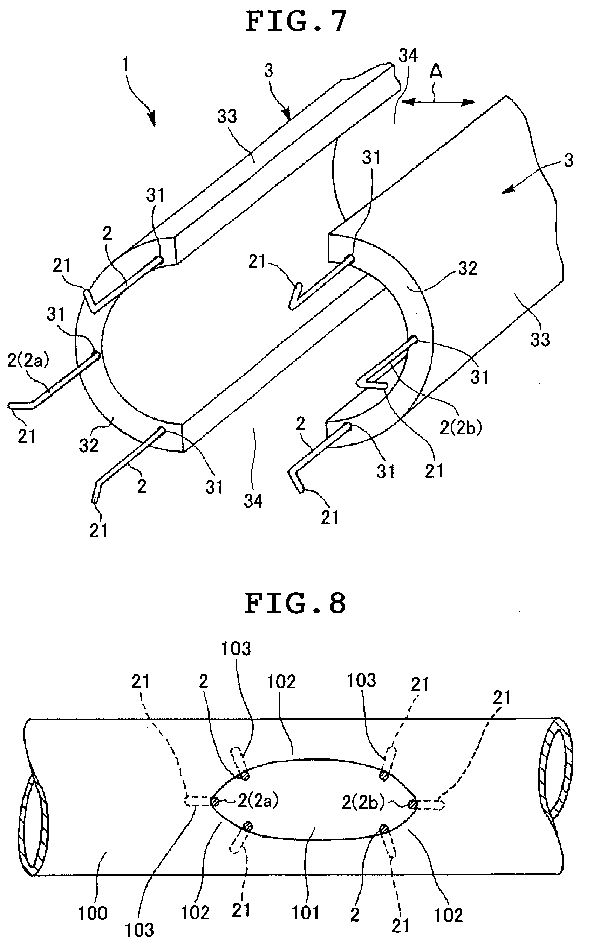

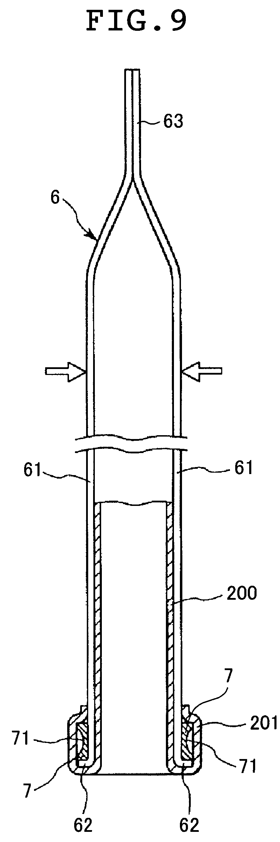

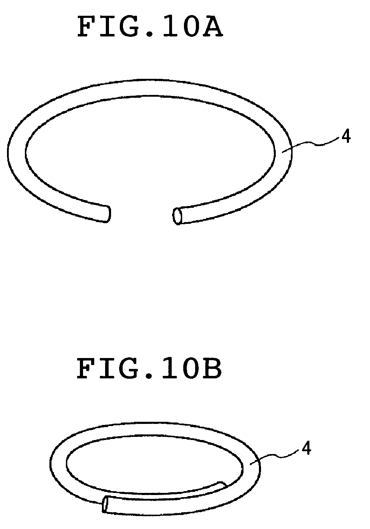

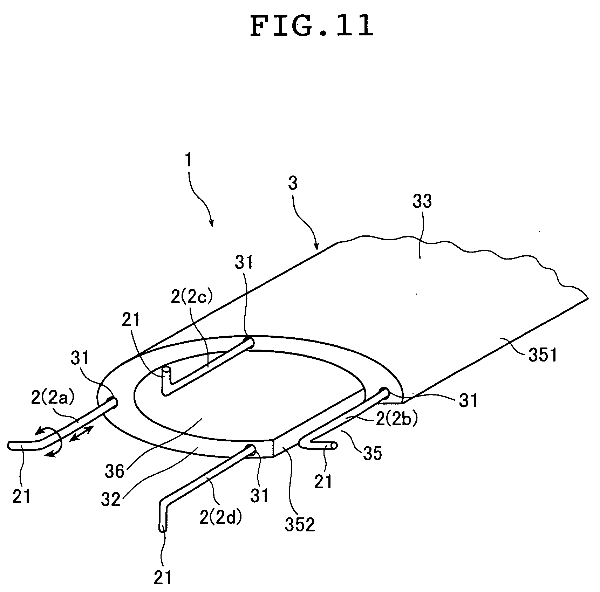

Figs. 1 to 6 each are a longitudinal cross sectional view showing a structure of a blood vessel connecting instrument according to the present invention and a using procedure thereof. Fig. 7 is a perspective view showing a structure of a distal end portion of the blood vessel connecting instrument according to the present invention. Fig. 8 is a plan view showing how engaging members in the blood vessel connecting instrument according to the present invention are attached to an opening (a dissected portion) of a blood vessel. Fig. 9 is a partial sectional side view showing a structure of a blood vessel supporting member and a receiving member in the blood vessel connecting instrument according to the present invention. Figs. 10A and 10B each are a perspective view showing a structure of a fastening member in the blood vessel connecting instrument according to the present invention. Fig. 11 is a perspective view showing a distal end portion in another structure of a holding member in the blood vessel connecting instrument according to the present invention. In the following explanation, as for each constitution of the blood vessel connecting instrument, the side closer to a blood vessel is referred to as "distal end" and opposite side thereof is referred to as "proximal end", respectively.

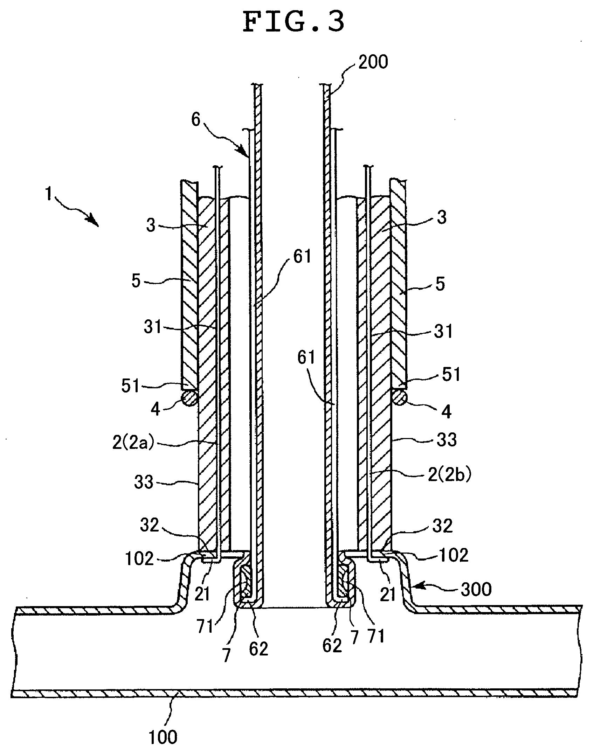

Referring to the attached figures, particularly Fig. 3 - Fig. 5, a blood vessel connecting instrument 1 in accordance with a first embodiment of the present invention is a blood vessel connecting instrument for connecting an opening (i.e., opening formed by being dissected by means of a knife or the like) 101 on a side portion of a first blood vessel 100 and an end portion 201 of a second blood vessel 200 to each other. The blood vessel connecting instrument 1 is provided with a plurality of engaging members 2 each of which is constituted by a linear body, a pair of holding members 3 (which are divided in two pieces), a fastening member 4, a pressing member 5 which operates for pressing and moving the fastening member 4, a blood vessel supporting member 6 which supports the second blood vessel 200, and a ring-shaped receiving member 7 being attached to the second blood vessel 200. Hereinafter, the structure of each component is illustrated in detail.

As shown in Fig. 7, each holding member 3 comprises an elongated member having a semi-circular cross section. In the holding members 3, small holes 31 wherein start from distal end surfaces 32 thereof and extend inside thereof in the longitudinal direction are formed. The number of the small holes 31 formed on both the holding members 3 is the same as that of the engaging members 2 constituted by linear bodies. An engaging member 2 is inserted into a small hole 31. In Fig. 7, three small holes 31 are formed in each holding member 3, an engaging member 2 is inserted into each small hole 31, thus six engaging members in total are inserted into the small holes 31 of both the holding members 3. However, at least one small hole 31 need only be formed in each holding member 31. Thus, at least two engaging members 2 suffice.

The engaging member 2 is movable in the small hole 31 in the longitudinal direction of the holding member 3. The engaging member 2 can also be rotated on the longitudinal axis of the small hole 31. This enables each engaging portion 21 formed on the distal end portion of the engaging member 2 to assume a posture in which the engaging member 2 projects outwardly from a central portion of the opening 101 (cf. Figs. 7 and 8). The respective engaging members 2 in the illustrated structure are provided substantially parallel to each other but this is not the sole case of the present invention. The respective engaging members 2 may be slanted inside so that they can be closer to each other toward the distal end (they can be directed toward the central line of the blood vessel connecting instrument 1).

The distal end portion of the engaging member 2 is projected from a distal end surface (clipping portion) 32 of the holding member 3. The distal end of the engaging member 2 has an engaging portion (hook) 21 formed by bending (folding) the distal end portion of the linear body substantially at right angles. The engaging portion 21 can be engaged with a peripheral portion 102 of the opening 101 formed in the side portion of the first blood vessel 100 from the inside.

As indicated in Fig. 7, both the holding members 3 are placed with their curved concave surfaces facing each other. As indicated by the arrow A in Fig. 7, both the holding members 3 are movable in the direction in which they come closer to or remoter from each other. This makes it possible to vary the distance between the engaging members 2 provided in the one holding member 3 and the engaging members 2 provided in the other holding member 3. As a result of this, the engaging portions 21 can be engaged under optimal conditions depending on the size, length (dissection length) and so forth of the opening 101.

The present invention may have a means for locking (preventing) movement of the holding members 3 in the directions indicated by the arrow A to keep the distance between the holding members constant. Alternatively, the present invention may be configured to prevent such movement.

Slits 34 extending in the longitudinal direction of the holding members 3 are formed between both the holding members 3 on the upper and lower sides in Fig. 7, respectively. In other words, in the blood vessel connecting instrument 1 according to this embodiment, the space between the two holding members 3 which are placed so that their curved concave surfaces face each other forms the slits 34. The second blood vessel 200 can also be inserted through the slits 34 inside the holding members 3. Since the slits 34 are constituted by the space between the holding members 3, the distal end surfaces 32 of the holding members 3 are open, which facilitates the operation of inserting the second blood vessel 200 through the slits 34 inside the holding members 3.

When the holding members 3 are configured to be movable in the directions indicated by the arrow A, the width of the slits 34 varies with their movement. On the other hand, when the holding members 3 are not configured to be movable in the directions indicated by the arrow A, the width of the slits 34 is constant.

The number of the engaging members 2 is not particularly limited, but is preferably four to six. Two of them that are most spaced from each other are selected from among the engaging members 2 provided in one of the holding members 3 and those provided in the other. The blood vessel connecting instrument 1 of the present invention can be used such that an imaginary straight line connecting the two engaging members 2 is directed in the longitudinal direction of the opening 101 in the first blood vessel 100. Referring to Fig. 8, the blood vessel connecting instrument 1 has six engaging members 2 in total. An engaging member 2a in the center of the three engaging members 2 arranged in one holding member 3 and an engaging member 2b in the center of the three engaging members 2 arranged in the other holding member 3 are most spaced from each other. The blood vessel connecting instrument 1 is used such that an imaginary straight line connecting the engaging members 2a and 2b is directed in the longitudinal direction (in the direction of dissection) of the opening 101 in the first blood vessel 100. As a result of this, the engaging portions 21 can be engaged under optimal conditions depending on the size, length (dissection length) and so forth of the opening 101.

The distal end surface 32 of the holding member 3 forms between it and the engaging portion 21 a clipping portion that clips the peripheral portion 102 of the opening 101. That is, by relatively moving the engaging members 2 in the direction toward the proximal end with respect to the holding member 3, the peripheral portion 102 of the opening 101 can be clipped between the engaging portion 21 and the distal end surface 32 of the holding member 3. Thus, the holding member 3 constitutes a holding means for holding the state in which the engaging portion 21 is engaged with the peripheral portion 102 of the opening 101 formed on the side portion of the first blood vessel 100.

As a material for the holding members 3, for example, various metal materials such as stainless steel, titanium or titanium alloys, and aluminum, and relatively hard resin materials such as polycarbonate, acrylic resin, polytetrafluoroethylene (Teflon resin), polyethylene, and polypropylene can be used.

Further, as the material for the engaging members 2, for example, various metal materials such as stainless steel, superelastic alloys (Ni-Ti based alloys), and titanium or titanium alloys, and carbon fiber. Among these, superelastic alloys and titanium are particularly preferred in terms of excellent compatibility with blood vessels in organisms.

The wire diameter of the engaging member 2 is not particularly limited. Usually, it is preferably about 0.05 mm to about 1.0 mm, more preferably about 0.2 mm to about 0.5 mm.

The length of the engaging portion (hook) 21 is not particularly limited. Usually, it is preferably about 0.1 mm to about 2.0 mm, more preferably about 0.3 mm to about 1.0 mm. Within such a range of length, the engagement of the engaging members 2 with the peripheral portion 102 of the opening 101 can be ensured and detachment from the peripheral portion 102 of the opening 101 can be readily manipulated.

It should be noted that in the present invention the shape, structure, number, layout of arrangement and so forth of the engaging members 2 and holding members 3 are not limited to those shown in the drawings.

A fastening member 4 is placed on the outer peripheral surfaces 33 (curved convex surfaces) thereof. The fastening member 4 is ring-shaped (partially cut out C-shaped ring and its diameter can be increased and decreased in the radial direction) as shown in Fig. 10A. The fastening member 4 holds both the holding members 3 therein with its inner peripheral surface being held in contact with the outer peripheral surfaces 33 of both the holding members 3. The fastening member 4 comprises an elastic material and its diameter can be increased or decreased in the radial direction. The elastic material includes, for example, metal materials such as stainless steel, titanium or titanium alloys, and superelastic alloys (Ni-Ti based alloys).

The fastening member 4 in a natural state (in a state where no external force is applied thereto) is constricted to be in a state of a reduced diameter (in a partially overlapped state) as shown in Fig. 10B. Application of a force to the fastening member 4 so as to extend it, it is extended into a state of an increased diameter as shown in Fig. 10A. However, the fastening member 4 is not limited to the ring with a cutout shown in Fig. 10A, it may be a ring of which the diameter can be increased or decreased in the radial direction.

The fastening member 4 in a state of an increased diameter (the state shown in Fig. 10A) is set around the outer peripheral surfaces 33 (curved convex surfaces) of both the holding members 3 which are arranged with their curved concave surfaces 33 facing each other (cf. Fig. 1).

Also, a pressing member 5 is placed on the outer peripheral surfaces 33 of both the holding members 3. The pressing member 5 for pressing and moving the fastening member 4 is arranged as a moving means for moving the fastening member 4 to the fixing position 300 in which the first blood vessel 100 is fixed to the second blood vessel 200.

The pressing member 5 is arranged so as to be relatively movable in the longitudinal direction with respect to the holding members 3. On the distal end of the pressing member 5, a pressing portion 51 for pressing the fastening member 4 is formed.

Moving the pressing member 5 in the direction toward the distal ends of the holding members 3 in the state as shown in Fig. 1, for example, to press the fastening member 4 causes the fastening member 4 to move in the direction toward the distal ends of the holding members 3, while sliding on the outer peripheral surfaces 33 of both the holding members 3 with the state of an increased diameter maintained. Therefore, the outer peripheral surfaces 33 of both the holding members 3 constitute a guide surface (guide portion) for guiding the fastening member 4 to the fixing position 300 in which the first blood vessel 100 is fixed to the second blood vessel 200.

When the blood vessel connecting instrument 1 of the present invention is used to connect the end portion 201 of the second blood vessel 200 to the opening 101 on the side portion of the first blood vessel 100, a blood vessel supporting member 6 shown in Fig. 9 supports the second blood vessel 200 in the state in which its end is opened. On this occasion, a ring-shaped receiving member 7 for receiving fastening force given by the fastening member 4 is attached to the end portion 201 of the second blood vessel 200, so that the second blood 200 is supported by the receiving member 7 and the blood vessel supporting member 6.

The blood vessel supporting member 6 has a pair of arm portions 61 whose proximal ends 63 are fixed to each other. Both the arm portions 61 comprise an elastic material, for example, a metal material such as stainless steel or a hard resin. The distal ends 62 of the arm portions 61 can become closer to or spaced from each other as a result of elastic deformation.

The distal end portions (bent portions) 62 of both the arm portions 61 are respectively bent in the vertical direction with respect to the longitudinal direction of the arm portions 61 and in the direction in which the distal end 62 of one arm portion 61 is spaced from the distal end 62 of the other arm portion 61. That is, the distal end portions 62 of both the arm portions 61 are respectively bent outwardly and are engaged with the receiving member 7 so that the dropping off the receiving member 7 can be prevented.

The receiving member 7 may be formed from various metal materials such as stainless steel, titanium or titanium alloys, relatively hard resin materials such as polycarbonate, acrylic resin, polytetrafluoroethylene (Teflon resin), polyethylene, and polypropylene, and ceramic materials such as apatite.

The inner diameter of the receiving member 7 is preferably substantially identical with the outer diameter of the second blood vessel 200. A groove 71 oriented in the longitudinal direction of the receiving member 7 is formed on the entire outer periphery of the receiving member 7. The groove 71 has an arcuately curved concave surface. Provision of the groove 71 having such a construction can further ensure clipping and fixing the wall of the first blood vessel 100 to the wall of the second blood vessel 200 when fastened by means of the fastening member 4, so that dislocation, breakaway disengagement and so forth of the fastening member 4 can be prevented.

In particular, since the groove 71 has an arcuately curved concave surface, the receiving member 7 can establish an excellent contact with the walls of blood vessels so that blood leakage and so forth can be securely prevented.

In this embodiment, the walls of the first and second blood vessels are clipped between the fastening member 4 and the receiving member 7 in a state in which they overlap each other, whereby they are securely fixed. In particular, the fastening member 4 and the receiving member 7 which are both ring-shaped can clip therebetween the peripheral portion 102 of the opening 101 of the first blood vessel 100 and the end portion 201 of the second blood vessel 200 over their whole periphery with substantially identical force. Therefore, this embodiment is advantageous in less injured blood vessels, higher connection strength, no gap generation in the connecting portion between both the blood vessels, and secure prevention of blood leakage or the like.

Next, the procedure for using the blood vessel connecting instrument 1 according to the present invention will be illustrated with reference to the case of connecting blood vessels to each other (anastomosis) in living body. However, the first blood vessel 100 and the second blood vessel 200 are not limited to blood vessels from living body but may be artificial blood vessels.

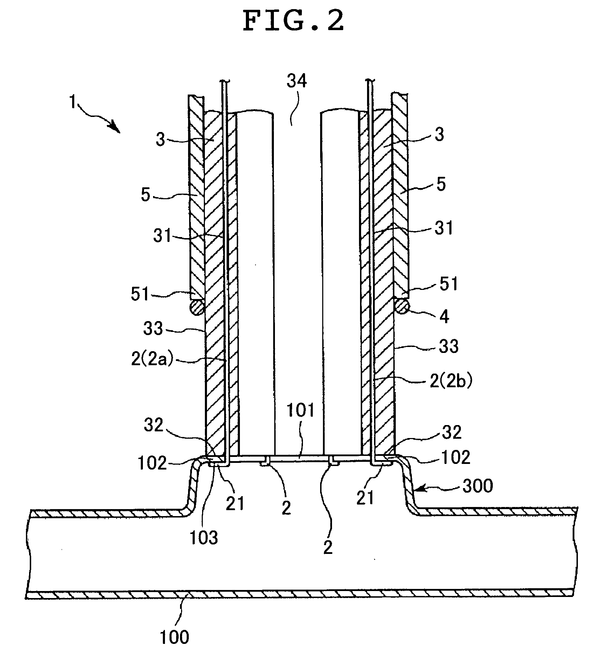

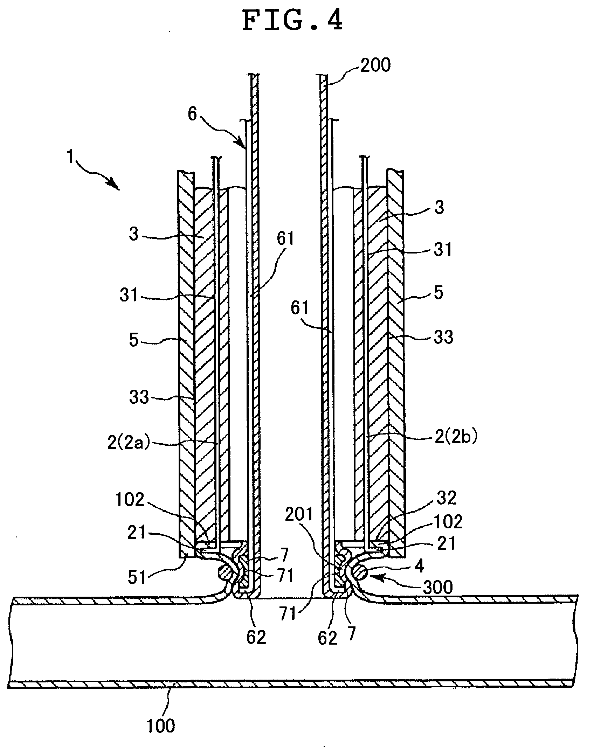

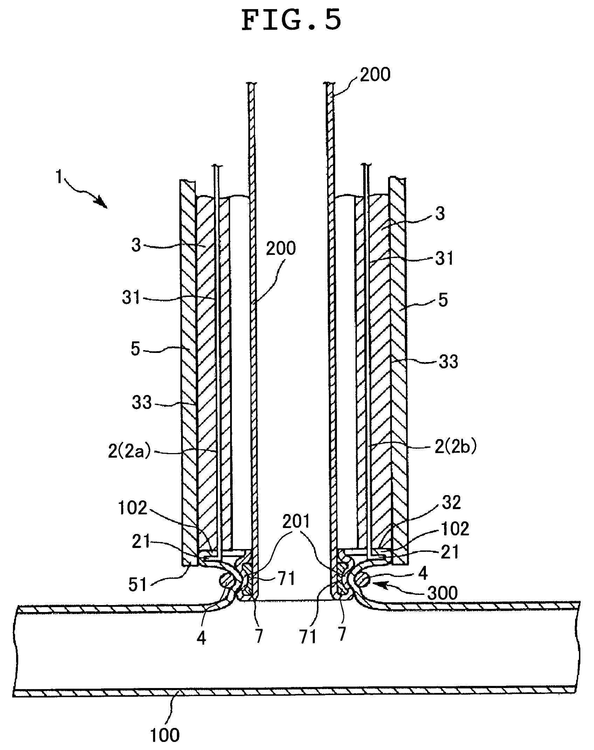

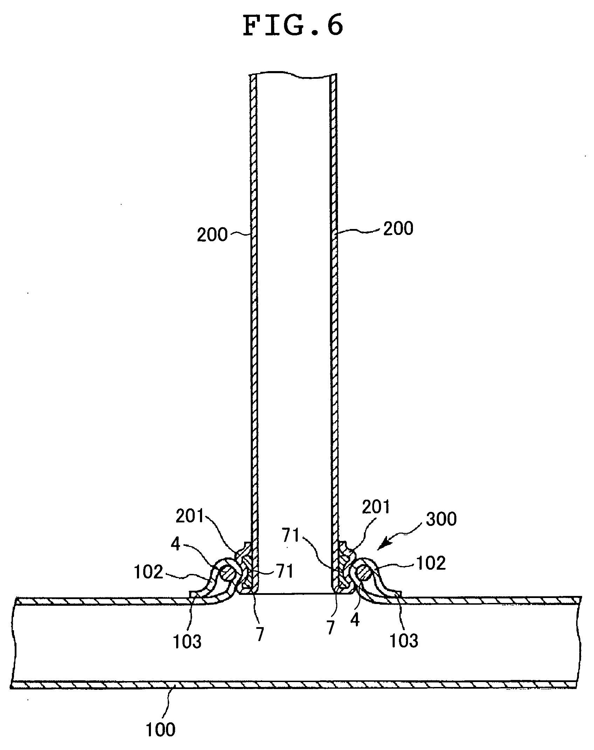

An exemplary method for using the blood vessel connecting instrument 1 will be illustrated below.
[1] A side portion of the first blood vessel (for example, a bypass blood vessel such as a coronary artery) 100 is partially dissected in the longitudinal direction to form a linear opening 101.
[2] The blood vessel connecting instrument 1 is arranged in a state where each engaging member 2 is projected from the distal end surface 32 of the holding member 3 by a sufficient length. Then, the distal end portion of each engaging member 2 is inserted in the opening 101 and the engaging portion 21 thereof is abutted against the peripheral portion 102 (inner side of the blood vessel wall) of the opening 101 (cf. Figs. 1 and 8). On this occasion, each engaging portion 21 is adjusted so as to have a posture of extending radially outwardly from the center of the opening 101. Further, by adjusting the distance between both the holding members 3, the engaging members 2a and 2b are arranged respectively so as to be positioned on both the ends of the opening 101 in the longitudinal direction thereof.
[3] By pulling each engaging member 2 in the direction toward its proximal end or moving the holding members 3 in the direction toward its distal end, the blood vessel connecting instrument 1 is moved in the direction toward proximal end thereof while clipping the blood vessel wall of the first blood vessel 100 by the engaging portion 21 and the distal end surface (clipping portion) 32 of the holding member 3 to partially elevate the side portion of the first blood vessel 100 (cf. Fig. 2). A protruded portion of the first blood vessel 100 formed by the elevation serves as the fixing position (second position) 300 for fixing the first blood vessel 100 and the second blood vessel 200 to each other.
[4] On the other hand, the second blood vessel (for example, a blood vessel to be bypassed, such as an internal thoracic artery) 200 is supported by the blood vessel supporting member 6. This is achieved by the following method.
   Both the arm portions 61 of the blood vessel supporting member 6 are pressed in the directions indicated by the arrows in Fig. 9 to temporarily make them closer to each other so that their distal end portions 62 can be inserted inside the receiving member 7 and then the pressing force is released. This generates a force that makes both the arm portions 61 to be spaced from each other so that both the arm portions 61 can be abutted against the inner surface of the receiving member 7 and at the same time the distal ends 62 of the arm portions 61 can be engaged with the distal end of the receiving member 7 and be held. In this state, the second blood vessel 200 is inserted between both the arm portions 61 and its distal end portion (end portion) 201 is turned inside out to be reversed it. This is used to cover the receiving member 7 (cf. Fig. 9).
[5] The second blood vessel 200 supported by the blood vessel supporting member 6 is inserted between both the holding members 3 and positioned such that the receiving member 7 covered with the second blood vessel 200 is placed at the fixing position 300 (cf. Fig. 3). The second blood vessel 200 may be inserted between both the holding members 3 (inside the holding members) by either a method of inserting it from the proximal end side of the holding members 3 or a method of inserting it through the slits 34.
[6] The pressing member 5 is moved in the direction toward the distal end portion relative to the holding members 3 to press the fastening member 4. As a result of this, the fastening member 4 moves in the direction toward the distal end of the holding members 3, while sliding on the outer peripheral surfaces 33 of both the holding members 3, with the state of an increased diameter (the state shown in Fig. 10A) maintained. When the fastening member 4 exceeds the distal ends of the holding members 3, the reaction force applied by the holding members 3 disappears. As a result, the fastening member 4 is constricted to have a reduced diameter due to its own contractile force (elastic force) as shown in Fig. 10B. This causes the overlapping portions (the fixing position 300) of the second blood vessel 200 and the first blood vessel 100, i.e., the overlapping portions of the end portion 201 of the second blood vessel 200 and the peripheral portion of the opening 101 of the first blood vessel 100, to be clipped between the fastening member 4 and the receiving member 7 over their entire periphery and fixed therebetween (cf. Fig. 4).
   The receiving member 7 has a groove 71 so that a firm and secure fixing and connection thereof can be achieved as described above. As a result, the dislocation or disengagement between the fastening member 4 and the receiving member 7 can be prevented and also excellent fluid seal can be obtained so that there occurs no blood leakage.
   The fixing position 300 described above is remoter from the opening 101 than the position 103 in which the engaging portion 21 of each engaging member 2 is engaged with the inner surface (endoderm) of the first blood vessel 1. That is, the distance between the fixing position 300 and the peripheral portion 102 of the opening 101 is greater than the distance between the peripheral portion 102 of the opening 101 and the position 103 where the engaging portion 21 of each engaging member 2 is engaged with the inner surface (endoderm) of the first blood vessel 100. This gives rise to the following effects.
   That is, the position 103 against which the engaging portion 21 on the inner surface of the first blood vessel 100 is abutted has the possibility of forming thrombi when it is brought in contact with blood flow due to a wound or the like. However, the position 103 is positioned outside the connection site 300 (the fixing position 300) of the blood vessels because the distance of the connection site 300 from the peripheral portion 102 of the opening 101 is greater than that of the position 103 from the peripheral portion 102 of the opening 101 so that it does not contact the blood flow (cf. Figs. 6 and 8). Therefore, formation of thrombi can be suppressed.
[7] Then, the blood vessel supporting member 6 is detached (cf. Fig. 5) by the following method. That is, while pressing both the arm portions 61 in the directions as indicated by the arrows in Fig. 9 so that they can get closer to each other, the distal ends 62 of both the arm portions 61 are withdrawn from inside the receiving member 7 and both the arm portions 61 are pulled in the direction toward the proximal end thereof.
[8] Finally, the blood vessel connecting instrument 1 is detached and the operation of connection (anastomosis) of the first blood vessel 100 and the second blood vessel 200 with each other is completed (cf. Fig. 6).

The operation of detaching the blood vessel connecting instrument 1 can be performed as follows. First, both the holding members 3 are moved in the direction toward the proximal end thereof with respect to each engaging member 2 to release the clipping of the blood vessel wall at the peripheral portion 102 of the opening 101. Then, the engaging portion 21 of each engaging member 2 is detached from the peripheral portion 102 of the opening 101. Thereafter, the blood vessel connecting instrument 1 is moved in the direction toward the proximal end thereof.

The operation of detaching the engaging portion 21 from the peripheral portion 102 of the opening 101 is performed preferably sequentially for each engaging member 2. This process can be performed with ease, for example, by rotating the engaging member 2 so that the engaging portion 21 can be directed toward the center of the opening 101.

The first blood vessel 100 and the second blood vessel 200 thus connected (anastomosed) through the steps as described above are connected to each other firmly and securely and are prevented or suppressed from forming thrombi in the vicinity of the connected portion so that good conditions for patients can be maintained for a long term.

Fig. 11 shows a structure of a blood vessel connecting instrument according to a second embodiment of the present invention which is different from the first embodiment in the structure of the holding member. Distinctions from the above-mentioned holding member about its structure and operation will be mainly described below and description of the similar matters will be omitted.

A holding member 3 shown in Fig. 11 is a hollow tube and a cutout extending in the longitudinal direction is formed in a part of a side surface 33 in the peripheral direction. The cutout constitutes a slit 35. A lumen 36 of the holding member 3 is substantially oval (or elliptical) in shape. This shape corresponds to that of an opening 101 of a first blood vessel 100 and enables easy and secure engagement of an engaging portion 21 of each engaging member 21 with a peripheral portion 102. However, there are no particular limitations on the shape of a distal end surface 32 of the holding member 3 and the distal end surface 32 may have for example any other shapes such as a circular shape.

In (the wall of) the holding member 3 are formed small holes 31 extending from the distal end surface 32 in the longitudinal direction. An engaging member 2 constituted by a linear body is inserted into each of the small holes 31. Referring to Fig. 11, four engaging members 2 are inserted into the small holes 31 formed to extend from the distal end surface 32 of the holding member 3 and spaced from each other at substantially the same intervals along the peripheral direction of the holding member 3. More specifically, when the oval (elliptical) lumen 36 is seen from the distal end surface 32 of the holding member 3, engaging members 2a and 2b are provided on both the ends of the distal end surface 32 in the long-diameter direction (horizontal direction in Fig. 11), respectively, and engaging members 2c and 2d are provided on both the ends thereof in the short-diameter direction (vertical direction in Fig. 11), respectively.

As described above, the cutout constituting the slit 35 is formed in a part of the holding member 3 in the peripheral direction. When seen from the peripheral direction of the holding member in the illustrated structure, the slit 35 is formed in a position between the engaging members 2b and 2d adjacent to each other. The slit 35 extends along the longitudinal direction of the holding member 3 as in the slit of the holding member in the first embodiment described above, and the distal end of the slit 35 opens on the distal end surface 32 of the holding member 3. The slit 35 may be formed over the whole length or up to some midpoint of the holding member 3 in the longitudinal direction.

The second blood vessel 200 can pass through the slit 35. In other words, the second blood vessel 200 can be introduced through the slit 35 into the lumen 36 of the holding member 3 when connected with (anatomosed into) the first blood vessel 100.

The width of the slit 35 in the holding member 3 shown in Fig. 11 (length in the peripheral direction of the holding member 3) has no particular limitations as long as the slit 35 has a sufficient width to pass the second blood vessel 200 therethrough. However, the width is preferably one-tenth to one-fourth, more preferably one-sixth to one-fourth of the whole peripheral length of the holding member 3. Too small width of the slit (cutout) 35 makes difficult the operation of passing the second blood vessel 200 through the slit 35, whereas too large width of the slit 35 makes it difficult to provide four or more engaging members 2 in the peripheral direction at the same intervals.

The width of the slit 35 may be constant or varied over the whole length of the slit (cutout) 35. For example, in the vicinity of the distal end portion of the holding member 3, the slit 35 may have a portion at which its width decreases or increases gradually toward the distal end.

Further, edges 351, 352 of the slit 35 are preferably chamfered or rounded off, whereby the second blood vessel 200 passing through the slit 35 is surely prevented from being injured by the edges 351, 352 of the slit (cutout) 35.

The method for using the blood vessel connecting instrument 1 having the above-mentioned holding member 3 is substantially the same as that in the first embodiment except that the second blood vessel 200 is inserted through the slit 35 into the lumen 36 of the holding member 3 in the step [5] described above.

The holding member 3 shown in Fig. 11 eliminates the necessity for the mechanism by which the distance between the holding members 3 as shown in Fig. 7 can be changed and hence has an effect of providing the blood vessel connecting instrument 1 with a more simplified structure.

Although the blood vessel connecting instrument according to the present invention was explained based on the embodiments illustrated in the appended drawings, the construction of respective components of the instrument according to the present invention may be replaced by any desired construction that can exhibit the same or similar functions.

Also, in the embodiments described above, the blood vessel connecting instrument 1 comprises engaging members 2, one or more holding members 3, a fastening member 4, a pressing member 5, a blood vessel supporting member 6, and a receiving member 7. However, the blood vessel connecting instrument 1 of the present invention need only have at least engaging members 2 and one or more holding members 3 among these components and all or a part of the other components may be omitted.

For example, the first blood vessel may be connected to the second blood vessel by suturing instead of using the ring-shaped fastening member 4.

Each component may be used either in an integrated state or a connected state or as independent members. Especially, the fastening member 4 and the receiving member 7 are parts that are used anew for every operation. Accordingly, they may be separate members independent of the body of the blood vessel connecting instrument 1.

As another construction, in the case where the second blood vessel 200 is an artificial blood vessel, the receiving member 7 may be fixed to or integrated into the second blood vessel 200 in advance.

As explained above, according to the present invention, connection (anastomosis) of blood vessels to each other can be realized readily, securely and quickly so that loads on patients can be reduced.

In particular, in the case of connection of blood vessels of small diameters, work under narrow field of vision, works under heart beating and so forth that have been conventionally difficult, quick and accurate connection of blood vessels is possible according to the present invention.

Furthermore, according to the present invention, the site on the inner surface of the blood vessel where the engaging portion is engaged is not exposed to blood flow. In addition, after the anastomosis, no foreign matter is present in the portion that contacts blood, so that there is little fear that thrombin formation can occur.

Furthermore, because remote manipulation is possible as well as the operation is easy, the present invention can be applied to, for example, operation of performing blood vessel connection of heart without thoracotomy (operation under an endoscopy). Therefore, the present invention finds a wide range of application.

Also, the blood vessel connecting instrument of the present invention find a wide range of application with respect to the type of blood vessel to be connected. Using them, not only graft blood vessels such as great saphenous vein with both ends but also pedicle graft such as internal thoracic artery can be connected with ease.

## Claims

1. A blood vessel connecting instrument for connecting an end portion of a second blood vessel to a side portion of a first blood vessel, comprising:
a plurality of engaging members each having an engaging portion which can be engaged with a peripheral portion of an opening formed in the side portion of the first blood vessel from inside; and
a holding means for holding the state in which the engaging portion is engaged with the peripheral portion of the opening in the first blood vessel.

2. A blood vessel connecting instrument according to claim 1, which further includes a fastening member for securely tightening the end portion of the second blood vessel overlaid on the vicinity of the peripheral portion of the opening in the first blood vessel.

3. A blood vessel connecting instrument according to claim 2, wherein the fastening member is ring-shaped.

4. A blood vessel connecting instrument according to claim 2 or 3, wherein the fastening member has a variable diameter.

5. A blood vessel connecting instrument according to any one of claims 2 to 4, which further includes a receiving member for receiving fastening force applied by the fastening member.

6. A blood vessel connecting instrument for connecting an end portion of a second blood vessel to a side portion of a first blood vessel, comprising:
a plurality of engaging members each having an engaging portion which can be engaged with a peripheral portion of an opening formed in the side portion of the first blood vessel from inside;
a holding means for holding the state in which the engaging portion is engaged with the peripheral portion of the opening in the first blood vessel;
a ring-shaped fastening member; and
a receiving member for receiving fastening force applied by the fastening member,
wherein the end portion of the second blood vessel is overlaid on the vicinity of the peripheral portion of the opening formed in the first blood vessel, and the overlapping portion of the first blood vessel and the second blood vessel is fastened by the fastening member and the receiving member to connect the end portion of the second blood vessel to the side portion of the first blood vessel.

7. A blood vessel connecting instrument according to claim 5 or 6, wherein the receiving member is ring-shaped.

8. A blood vessel connecting instrument according to claim 7, wherein the receiving member has a groove on its outer periphery.

9. A blood vessel connecting instrument according to any one of claims 5 to 8, which further includes a blood vessel supporting member for supporting the second blood vessel together with the receiving member.

10. A blood vessel connecting instrument according to claim 9, wherein the blood vessel supporting member has a pair of arm portions which can come closer to and spaced from each other.

11. A blood vessel connecting instrument according to any one of claims 2 to 10, which further includes a guide portion for guiding the fastening member to a fixing position in which the first blood vessel is fixed to the second blood vessel and a moving means for moving the fastening member to the fixing position.

12. A blood vessel connecting instrument according to claim 11, wherein the fixing position is remoter from the opening than a position in which the engaging portion is engaged with an inner surface of the first blood vessel.

13. A blood vessel connecting instrument according to any one of claims 1 to 12, wherein each of the engaging members comprises a linear body.

14. A blood vessel connecting instrument according to claim 13, wherein the engaging portion is formed by bending a distal end of the linear body.

15. A blood vessel connecting instrument according to claim 13 or 14, wherein the number of the engaging members is four to six.

16. A blood vessel connecting instrument according to any one of the claims 1 to 15, in the plurality of engaging members, at least two of the engaging members are variable with respect to the distance between one engaging member and another one.

17. A blood vessel connecting instrument according to any one of claims 1 to 16, wherein the holding means has a clipping portion so that the peripheral portion of the opening in the first blood vessel can be clipped between the clipping portion and the engaging portion.

18. A blood vessel connecting instrument according to claim 17, wherein the engaging members are arranged in the holding means so as to be movable in the longitudinal direction thereof.

19. A blood vessel connecting instrument according to claim 17 or 18, wherein the holding means has slits through which the second blood vessel can pass.

20. A blood vessel connecting instrument according to claim 19, wherein the holding means comprises a pair of elongated members each having a semi-circular (arch-shaped) cross section and space between the elongated members placed so that their curved concave surfaces face each other forms the slits.

21. A blood vessel connecting instrument according to claim 19, wherein the holding means is a hollow tube of which both ends are open and has a slit which is a cutout formed on a side surface of the tube.

22. A blood vessel connecting instrument according to claim 21, wherein the holding means has a lumen which has a substantially oval or elliptical shape.

23. A blood vessel connecting instrument according to claim 22, wherein the number of the engaging members provided in a distal end surface of the holding means is four, of which two are arranged in portions corresponding to both ends of the oval or elliptical shape in the long-diameter direction and the other two are arranged in portions corresponding to both ends thereof in the short-diameter direction.

24. A blood vessel connecting instrument according to any one of claims 21 to 23, wherein the slit has a width of one-tenth to one-fourth of the whole peripheral length of the holding means.

25. A blood vessel connecting instrument for connecting an end portion of a second blood vessel to a side portion of a first blood vessel, comprising:
a tubular hollow holding member having a slit which is formed in a part of a side surface of the holding member in the peripheral direction and through which the second blood vessel can pass;
a plurality of engaging members each of which comprises a linear body, is arranged in the holding member so as to be movable in the longitudinal direction thereof, and can be engaged with a peripheral portion of an opening formed in the side portion of the first blood vessel from inside;
a fastening member; and
a receiving member for receiving fastening force applied by the fastening member,
wherein the end portion of the second blood vessel is overlaid on the vicinity of the peripheral portion of the opening in the first blood vessel, and the overlapping portion of the first blood vessel and the second blood vessel is fastened by the fastening member and the receiving member to connect the end portion of the second blood vessel to the side portion of the first blood vessel.

26. A blood vessel connecting instrument according to claim 25, wherein the fastening member or the receiving member or both are ring-shaped.

27. A blood vessel connecting instrument according to claim 25 or 26, wherein the engaging members are inserted into small holes which start from a distal end surface of the holding member and extend in the holding member in the longitudinal direction thereof.

28. A blood vessel connecting instrument according to any one of claims 25 to 27, wherein the distal end surface of the holding member has a substantially oval or elliptical shape.

29. A blood vessel connecting instrument according to claim 28, wherein the number of the engaging members provided in the distal end surface of the holding member is four, of which two are arranged in portions corresponding to both ends of the oval or elliptical shape in the long-diameter direction and the other two are arranged in portions corresponding to both ends thereof in the short-diameter direction.

30. A blood vessel connecting instrument according to claim 29, wherein the slit is formed in a position between two engaging members adjacent to each other when seen from the peripheral direction of the holding member.

31. A blood vessel connecting instrument according to any one of claims 25 to 30, wherein the slit has a width of one-tenth to one-fourth of the whole peripheral length of the holding member.

32. A blood vessel connecting instrument according to any one of claims 25 to 31, wherein the slit opens on the distal end surface of the holding member.
